# EUROPEAN PATENT SPECIFICATION

(11) **EP 2 779 912 B1**
(45) Date of publication and mention of the grant of the patent: **27.12.2017**
(21) Application number: 12784633.5
(22) Date of filing: 16.11.2012
(51) Int. Cl.: A61B 17/04

(54) **A SURGICAL DEVICE**
CHIRURGISCHE VORRICHTUNG
DISPOSITIF CHIRURGICAL

(30) Priority: 18.11.2011 GB 201119925
(43) Date of publication of application: 24.09.2014
(73) Proprietor: NeoSurgical Limited, Dublin 24 (IE)
(72) Inventor: KEATING, Ronan, Dublin 24 (IE)
(74) Representative: Hanna Moore + Curley
(86) International application number: PCT/EP2012/072936
(87) International publication number: WO 2013/072517

(56) References cited:
- WO-A2-2006/111955
- US-A- 5 306 278
- US-A- 5 507 758
- US-A- 5 562 688
- US-A1- 2005 021 055

## Description

### Field of the Invention

The present invention relates to a surgical device and in particular to a laparoscopic surgical device configured to allow for delivery of suture to effect a closure of a wound. The invention relates in one configuration to a surgical device configured for cooperation with a surgical instrument, for example a trocar, and which on cooperation allows for the delivery of suture.

### Background

There are difficulties sometimes associated with closure of wound sites for example, trocar port sites in laparoscopic procedures. There are difficulties in particular in finding the fascia layer through which a suture must be passed to ensure good and adequate port site closure.

With deeper port sites, such as with an obese patient, it is often more difficult for the surgeon to gain deep access to the fascial layer to securely place a suture therein. In certain instances it may be necessary to cut open the wound to accurately place a suture fixation on the inner fascia layer. This is counter-productive as the potential for a hernia to occur is related to the incision size.

The consequences of inadequate closure may be serious. For example, the patient may be subject to an early or late onset hernia, bowel stricture and/or bleeding from the port site. All of these complications have varying associated morbidities up to and including fatalities in serious undetected bowel strictures. The rate of port site herniation is widely published to be up to 3% for the normal population and double this for the obese cohort.

Current trocar port closure offerings require the removal of the trocar in order to place the closure device and facilitate closure. The disadvantage with this approach is that when the trocar is removed the path through the abdominal wall layers is lost and finding the original path can be difficult and cause more damage at the wound site.

There are therefore a number of problems with current methods of trocar port site closure that need to be addressed, particularly for the obese patient.

US2005/0021055 describes surgical instruments and guides that use two passageways to deliver suture for closure of fascia and other tissue sites.

WO2006/111955 describes a device for wound suturing and hemostasis in the thoracic and abdominal wall.

US5507758 describes a surgical instrument that is capable of being used for closure of fascia and for all uses related to accurately passing suture material through a guide into tissue.

### Summary

These needs and others are addressed by a laparoscopic device in accordance with the present teaching which provides for deployment of a suture to enable port site closure subsequent to a laparoscopic surgical procedure.

In one aspect a surgical device is provided for operably coupling with a trocar or other body. The device comprises a leading edge, a trailing edge and a mating surface provided therebetween which operably is in intimate contact with an outer surface of the trocar or the other body as appropriate. The device may further comprises a handle which allows for application of a downward pivotal force to drive the leading edge downwardly along the outer surface of the trocar concurrently bringing the trailing edge towards and into contact with the outer surface of the trocar. Examples of other bodies include a finger or other digit of a user of the device.

In another aspect a surgical device is provided for coupling with a trocar or other solid body. The device comprises a leading edge, a trailing edge and a trocar mating surface provided therebetween which operably is in intimate contact with an outer surface of the trocar or other body. The device further comprises only one needle guide channel. The needle guide channel comprising an entry port for receiving a suture delivery device and an exit port from which the suture delivery device may be driven away from the device. In one aspect, a surgical device is provided for coupling with a trocar, the device comprises a leading edge, a trailing edge and a trocar mating surface provided therebetween which operably is in intimate contact with an outer surface of the trocar, the device further comprises a handle which allows for application of a downward force to present the trocar mating surface to the outer surface of the trocar, the handle having a length greater than the length of the trocar mating surface.

In a still further aspect, a surgical device is provided for operably coupling with a trocar, the device comprises a leading edge, a trailing edge and a trocar mating surface provided therebetween which operably contacts with an outer surface of the trocar. The device further comprises a handle which allows for application of a downward force to present the trocar mating surface to the outer surface of the trocar, the handle having an upper surface dimensioned to accommodate a user's thumb.

In one aspect a surgical device is provided for operably coupling with a trocar. The device comprising an arcuate mating surface which operably is in intimate contact with an outer surface of the trocar, the mating surface extending only partially about the trocar. The device further comprising a needle guide channel comprising an entry port for a needle driver to be presented to the device and an exit port from which suture which is coupled to a needle or an anchor may be driven using the needle driver out of and away from the device.

In one aspect a surgical device is provided for operably coupling with a trocar. The device comprising an arcuate mating surface which operably is in intimate contact with an outer surface of the trocar. The arcuate mating surface defines an arc subtended by less than 180 degrees. The device further comprising a single needle guide channel comprising an entry port for receiving a needle driver and an exit port from which the needle driver may be driven away from the device.

These and other features of the present teaching will be better understood with reference to the drawings which follow which are provided to assist in an understanding of the present teaching and are not to be construed as limiting in any fashion.

### Brief Description of the Drawings

The present teaching will now be described with reference to the accompanying drawings in which:
Figure 1A is an isometric view of a device in accordance with the present teaching.
Figure 1B is an side view of the device.
Figure 2 is a top view of the device of Figure 1.
Figure 3 is a perspective view from the side of the device of Figure 1 and 2.
Figure 4 is a view showing a mating surface of the device.
Figure 5A is a section view along the line A-A of Figure 4.
Figure 5B shows another arrangement whereby the arcuate mating surfaces define an arc subtended by at least 180 degrees.
Figure 5C show another configuration configured for receipt of a finger or other digit of a user to allow for deployment of the device at a wound site.
Figure 6 shows the device of Figures 1 to 5 in cooperation with an exemplary trocar.
Figure 7 is a side view of the devices from Figure 6 deployed in a section of abdomen shown as section through the line A-A of Figure 6.
Figure 8 shows the provision of two sutures on opposing sides of a surgical site post removal of the trocar.
Figure 9 shows a closed surgical site.
Figure 10A and Figure 10B show in schematic form a vector analysis of a closure mechanism provided in accordance with the present teaching and how it achieves a reduction in applied tension.

### Detailed description of the drawings

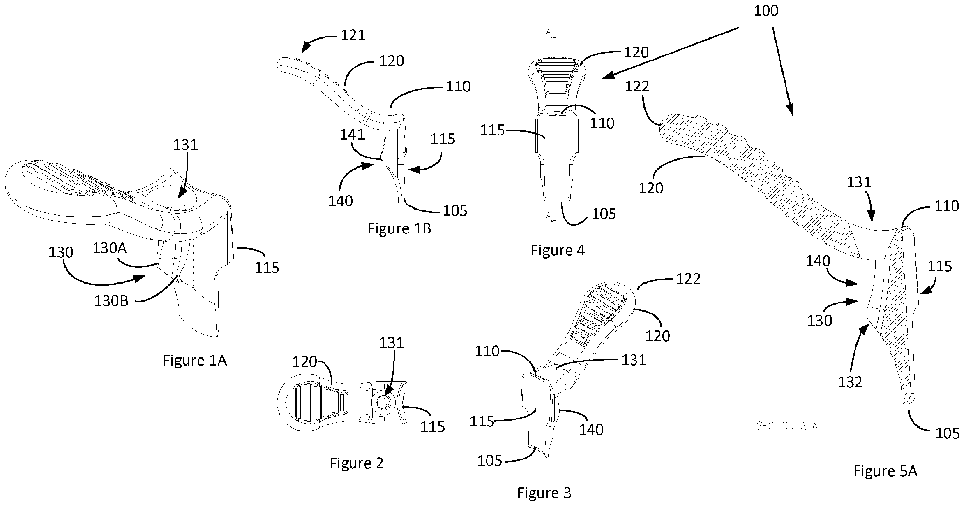

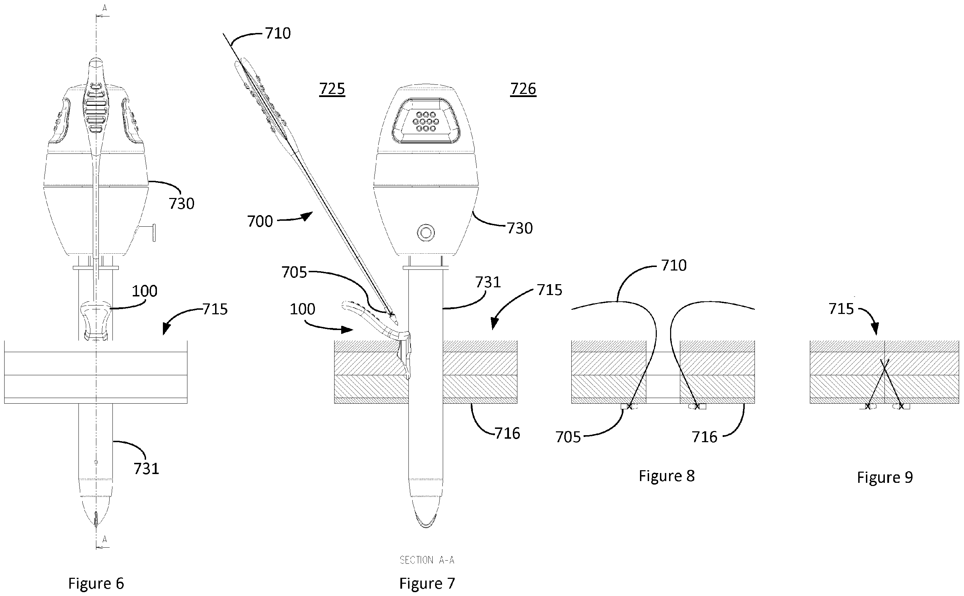

Figures 1 to 5 show an example of a surgical device 100 in accordance with the present teaching. As will be discussed with reference to Figures 6 and 7, the device is configured for operably coupling with a trocar and is usefully employed in the delivery of suture to allow for a closure of a surgical site post-surgery.

The device comprises a leading edge 105, a trailing edge 110 and a trocar mating surface 115 provided therebetween. The leading edge 105 is desirably chamfered having an internal radius to prevent catching on stepped or threaded trocar shafts.

The trocar mating surface is desirably an arcuate surface which operably is in intimate contact with an outer surface of the trocar or other solid body. Examples include a finger or other digit of a user deploying the device. In the configurations of Figures 1 through 5A, the angle subtended by the arc defining the arcuate surface is less than 180 degrees. In this way the arcuate surface extends only partially about the circumference of the trocar.

The device of these exemplary configurations further comprises a handle 120 which allows for application of a downward force to present the trocar mating surface 115 to the outer surface of the trocar. The handle comprises an upper surface 121 dimensioned to accommodate a user's thumb. This upper surface 121 may be textured or otherwise treated to increase the grip that a user experiences on contacting the handle. The texturing may be provided by overmoulding an elastomeric or other suitable material onto the body of the device. The lower surface 121B is desirably smoother than the upper surface so as to interact with the glove of the surgeon during use. The length of the handle is desirably longer than the length of the trocar mating surface 115. In certain configurations- not shown- the handle may have a length sufficient to be grasped within the palm of the user's hand. Such length facilitates and enables application of additional force onto the device. This may assist a user in locating the device in tight locations.

The device further comprises a needle guide channel 130 comprising an entry port 131 for a suture delivery device or needle driver to be presented to the device 100 and an exit port 132 from which the needle driver may be driven away from the device. As shown in Figure 1A, the needle guide channel 130 may be an open channel whereby at least a portion of the needle driver will extend beyond the needle guide channel during its delivery through the device. Two open walls 130A, 130B define the circumference of the guide channel 130 in the region below the entry port 131. This circumference is less than the circumference of a needle driver which is to be used with the device.

The needle entry port 131 is desirably provided as an off-centre hole which facilitates and eases presentation of the needle driver to the channel 130.

The needle guide channel further comprises a trocar mating surface proximal surface 130C which is that surface which is closest to the trocar mating surface 115. As a result of the arcuate path defined by the needle guide channel 130, the needle driver will be initially biased towards trocar mating surface proximal surface 130C and will adopt the path defined by that surface during its passage through the device. By providing an open needle guide channel, the profile of the device may be reduced to ensure that suture delivered through the needle guide channel will enter subcutaneously into the abdominal wall.

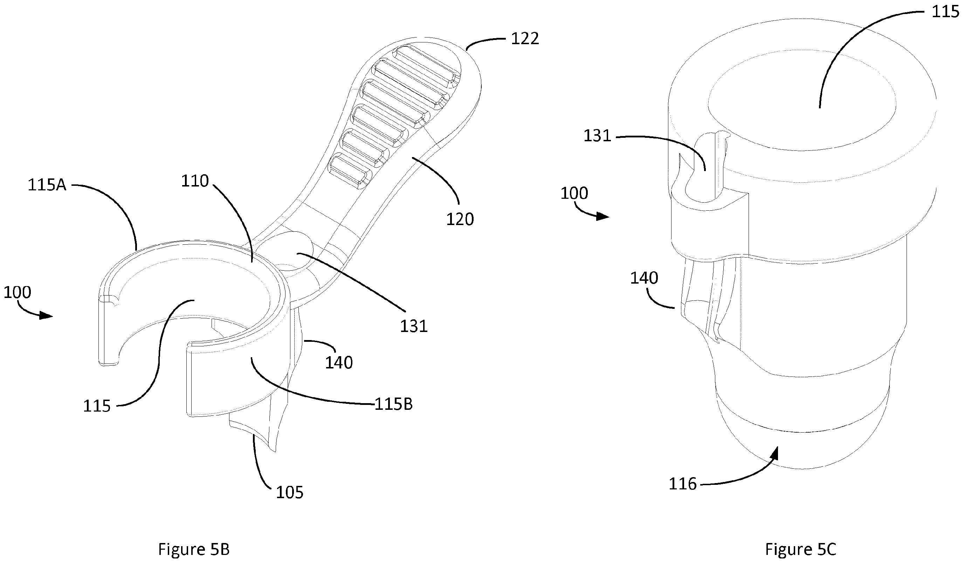

In the configurations heretofore described the arcuate mating surfaces define an arc subtended by less than 180 degrees. Figure 5B shows another arrangement whereby the arcuate mating surfaces define an arc subtended by at least 180 degrees. Two side walls 115A, 115B extend outwardly in a curved path so as to extend the contact area that the device will have with a trocar or other body. Such a configuration is particularly useful where there is a desire to provide an actual mating of the device with the trocar or other body. For example, the two side walls 115A, 115B may be resiliently biased towards one another such that on presentation of a trocar to the mating surface 115, the side walls 115A, 115B will displace outwardly so as to allow a receipt of the trocar within a volume defined by the arms. The side walls will then retract towards one another so as to actively engage with the trocar body- retaining the device in contact with the trocar. This is particularly useful in circumstances where it is desirably to keep the device and the trocar co-planar as movement of one will effect a corresponding movement of the other.

In certain configurations the side walls may be coupled to one another on an opposing side of the trocar to the location of the needle guide channels. In such a configuration, the device may be used with trocars of different diameters but by tensioning the two side walls relative to one another it is possible to provide an intimate fit between the device and the trocar irrespective of the diameter of the trocar.

Figure 5C shows another configuration configured for receipt of a finger or other digit of a user to allow for deployment of the device at a wound site. In this configuration the mating surface 115 extends to define a cap 116 with a closed end within which a finger may be located. Similar to a thimble, this allows the finger to be placed within the volume of the cap 116 so as to allow for placement of the device within a wound site. For extended wounds, the device may then be drawn along the wound walls delivering suture at determined locations as appropriate.

In the configurations described herein only one needle guide channel is provided which requires the delivery of suture to one side of the wound at a time. To close the wound, suture needs to be delivered to both sides so as to allow for a subsequent drawing of the wound together for closure. However by having only one needle guide channel the diameter of the device may be reduced which allows for application in wounds of varying diameter.

An example of use of the device with a needle driver 700 is shown in Figures 6 and 7. The needle driver 700 is configured to allow delivery of an anchor 705 which is coupled to suture 710. The anchor 705 is disposed on the tip of the needle driver and is presented through the abdominal wall 715 so as to allow for delivery of suture.

The entry port 131 is desirably defined within the handle 120 and is located proximal to the trailing edge 110. The length of the handle from the entry port to its tip 122 is desirably greater than the length of the trocar mating surface 115 from the trailing edge 110 to the leading edge 105.

The exit port 132 is provided on an outer surface 140 of the device 100. The outer surface 140 is on an opposite side of the device to the trocar mating surface 115. The needle guide channel 130 is desirably configured to taper outwardly such that the needle driver 700 presented through the guide channel will be directed into the abdominal wall 715 that is contacting the outer surface 140. In a first configuration the channel 130 provides a convex path relative to a longitudinal axis of the device such that a needle driver will initially be presented towards the trocar mating surface 115 on insertion through the entry port 131 and will then be displaced away from the trocar mating surface prior to exiting through the exit port 132.

The exit angle relative to the perpendicular is desirably a fixed angle which may be optimally configured between 5 and 30°, or more preferably between 10 and 20°. It will be appreciated that by orientating the tip 122 of the handle away from or towards the trocar 731 that the actual exit angle may be varied in situ by the surgeon. This is particularly advantageous in circumstances of use with obese patients.

As shown in Figure 8, by providing a needle guide channel, suture can be coupled to respective anchors and directed into the abdominal wall. The anchor is desirably biased inwardly through the guide channel 130 using the driver. The exit port 132 is desirably located such that the needle driver 700 will pass into the subcutaneous layer of the abdominal wall. Desirably application of continued downward pressure using the needle driver will cause the anchor 705 to then pass into the abdominal cavity, pulling suture with it.

On passage of the anchor 705 into the abdominal cavity it will desirably hang, suspended on its suture, after the anchor driver is removed. The orientation of the anchor will typically change orientation from a vertical disposition used in the deployment configuration to a horizontal configuration. This may be assisted by coupling the suture to an anchor at a mid-point of the anchor such that it will pivot relative to the coupling to change its orientation. Anchoring is effected by retracting the deployed suture. This causes the suture to be pulled back, tightening the anchor against the inner abdominal wall 716. As the orientation of the needle has changed, it will not tend to retreat back through the abdominal wall through the path it developed on penetration of the wall.

It will be appreciated that the closure device of the present teaching comprises only one needle guide channel. In use, a surgeon will typically require deployment of two or more sutures to facilitate the closure of a wound. The sutures are desirably deployed on either side of the wound- shown for example in Figure 8. To facilitate this deployment and as shown in Figure 7, the device 100 is typically presented to a first side 725 of a trocar 730. The trocar comprises a shaft 731. The shaft comprises a curved surface whose angle of curvature corresponds with the angle of curvature of the trocar mating surface 115 such that on presentation of one to the other the two surfaces mate with one another in an intimate contact. The surgeon may then deploy the suture through the needle guide channel to a first side of the wound - the same side 725 of the trocar 730 to which the needle driver 700 was presented to the device 100. The needle driver is then withdrawn and the device 100 separated from the deployed suture 710. The same process is then repeated on a second side 726 of the trocar 730. In this way deployment of suture to the two sides of the wound requires a presentation of the device twice to opposing sides of the trocar. This sequential presentation of the device is different to conventional closure systems whereby the device is presented only once to the trocar and then retained in position relative to the trocar during the deployment of suture about the wound site.

On completion of the surgical procedure, the deployed anchors and sutures may be used to effect a closure of the wound. The suture is provided on both sides of the surgical site - as shown in Figure 8. The surgeon may then tie a knot, and use the still tethered suture to effect a closure of the wound, as is shown in Figure 9. Desirably sutures and/or needle anchors are bio-absorbable so as to allow for their eventual dissolving after the procedure.

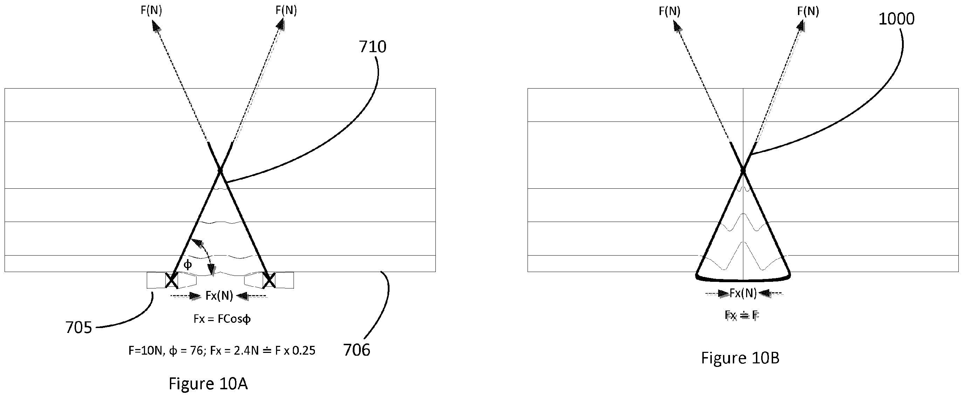

Figures 10A and 10B show an example of a vector analysis performed to illustrate a reduction in tension achievable by using a closure arrangement per the present teaching. The configuration per Figure 10A is as provided by the present teaching whereas that of Figure 10B is an example of a prior art closed loop technique using a single piece of suture 1000. In each arrangement the application of a force of for example 10 Newton, N, to each side of the open wound effects a drawing of the wound surfaces together to effect a closure of the wound. In the example of Figure 10A, where there is no closed loop, but rather two anchors provided on either side of the previously open wound, this application of a 10N force effects a closure force in the direction of Fx(N), using an example of an angle φ of 76 degrees it will be appreciated from fx= Cos φ = 10 Cos 76 that the force acting in the direction between the two anchors is about 2.4N. In a traditional closed loop configuration this force is about 10N. It will be appreciated that achieving closure with minimal force is a better surgical result and per the present teaching a 75% reduction is significant. This will help prevent over tightening which can be associated nerve pain and impaired healing.

The device 100 may be provided as a rigid element. In another configuration it may be formed from a flexible, for example elastomeric, material. This would allow the outer surface 140 or wall engaging portion to deform to adapt to the contours of the abdominal wall with which it engages.

The outer surface 140 is desirably contoured to define at least one displaced abutment surface 141 which in use will restrict movement of the device relative to the abdominal wall within which it is located. This surface 141 provides an anchoring of the device within the abdominal wall. The outer surface 140 preferably tapers inwardly towards the leading edge 105 of the device, this taper provides a tapered entry member 142 to ease insertion of the device into the abdominal wall. The leading edge 105 is desirably chamfered having an internal radius to prevent catching on stepped or threaded trocar shafts.

As was discussed above, an advantage of this device is that it may be located relative to a trocar that is already in position in the abdomen without removing the trocar. This is especially useful when applied to trocars off midline, where anchoring may not be necessary during the procedure, but closure is required.

While preferred arrangements have been described in an effort to assist in an understanding of the teaching of the present invention it will be appreciated that it is not intended to limit the present teaching to that described and modifications can be made without departing from the scope of the invention.

It will be appreciated that the exemplary arrangements or examples of devices have been described with reference to the Figures attached hereto. Where a feature or element is described with reference to one Figure, it will be understood that the feature or element could be used with or interchanged for features or elements described with reference to another Figure or example. The person of skill in the art, when reviewing the present teaching, will understand that it is not intended to limit the present teaching to the specifics of the illustrated exemplary arrangements as modifications can be made without departing from the scope of the present teaching.

The words comprises/comprising when used in this specification are to specify the presence of stated features, integers, steps or components but does not preclude the presence or addition of one or more other features, integers , steps, components or groups thereof.

## Claims

1. A surgical device (100) for coupling with a trocar (730) or other body, the device comprising:
a. a leading edge (105),
b. a trailing edge (110)
c. an arcuate mating surface (115) provided between the leading and trailing edges, the arcuate mating surface operably being in intimate contact with an outer surface of the trocar or other body,
d. only one needle guide channel (130), the needle guide channel comprising an entry port (131) for receiving a suture delivery device (700) and an exit port (132) from which the suture delivery device may be driven away from the device (100), the entry and exit ports for the needle guide channel being provided on the same side of the arcuate mating surface, the needle guide channel defining an arcuate path having a convex surface proximal to the arcuate mating surface such that the suture will exit away from the arcuate mating surface on being displaced out of the needle guide channel.

2. The device of claim 1 comprising a handle (120) which allows for application of a downward pivotal force to drive the leading edge downwardly along the outer surface of the trocar concurrently bringing the trailing edge towards and into contact with the outer surface of the trocar, optionally wherein the handle has a length greater than the length of the arcuate mating surface.

3. The device of claim 2 wherein the handle (120) comprises an upper surface dimensioned to accommodate a user's thumb.

4. The device of any preceding claim wherein the arcuate mating surface operably extends only partially about the trocar or other body, optionally wherein the arcuate mating surface defines an arc subtended by less than 180 degrees.

5. The device of any one of claims 1 to 3 wherein the arcuate mating surface defines an arc greater than or equal to 180 degrees.

6. The device of any preceding claim wherein the arcuate mating surface is configured to snap-engage with the trocar or other body.

7. The device of any one of claims 1 to 3 wherein the arcuate mating surface is configured to extend fully about the trocar or other body, optionally wherein the arcuate mating surface is configured to be tensioned against the trocar or other body.

8. The device of any preceding claim wherein the needle guide channel comprises an open portion within which a needle driver will operably extend out of the device during a passage of the needle driver through the device.

9. The device of any preceding claim wherein the arcuate mating surface defines a cap (116) within which a finger or other digit may be received.

10. The device of any preceding claim comprising an outer surface (140) contoured to define at least one displaced abutment surface (141) which in use will restrict movement of the device relative to the abdominal wall within which it is located.

11. The device of claim 10 wherein the abutment surface (140) provides an anchoring of the device within the abdominal wall.

12. The device of claim 10 wherein the outer surface tapers inwardly towards a leading edge of the device to provide a tapered entry member to ease insertion of the device into the abdominal wall.

13. The device of claim 12 wherein the leading edge is chamfered having an internal radius to prevent catching on stepped or threaded trocar shafts.

14. The device of any preceding claim wherein the entry port is off- centre relative to the handle to facilitate presentation of the suture delivery device to the needle guide channel.

15. The device of claim 2 wherein the handle has a length sufficient to allow location of the handle in the palm of a user's hand.

## Patentansprüche

1. Chirurgische Vorrichtung (100) zum Koppeln mit einem Trokar (730) oder einem anderen Körper, wobei die Vorrichtung Folgendes umfasst:
a. eine Vorderkante (105),
b. eine Hinterkante (110)
c. eine bogenförmige Passfläche (115), die zwischen der Vorderkante und der Hinterkante bereitgestellt ist, wobei sich die bogenförmige Passfläche betriebsfähig in engem Kontakt mit einer Außenfläche des Trokars oder des anderen Körpers befindet,
d. nur einen Nadelführungskanal (130), wobei der Nadelführungskanal eine Eintrittsöffnung (131) zum Aufnehmen einer Fadenzuführvorrichtung (700) und eine Austrittsöffnung (132), aus der die Fadenzuführvorrichtung von der Vorrichtung (100) weg getrieben werden kann, umfasst, wobei die Eintrittsöffnung und die Austrittsöffnung für den Nadelführungskanal auf derselben Seite der bogenförmigen Passfläche bereitgestellt sind, wobei der Nadelführungskanal einen bogenförmigen Pfad mit einer konvexen Oberfläche proximal zu der bogenförmigen Passfläche definiert, sodass der Faden von der bogenförmigen Passfläche weg austritt, wenn er aus dem Nadelführungskanal heraus verschoben wird.

2. Vorrichtung nach Anspruch 1, umfassend einen Griff (120), der das Ausüben einer Drehkraft nach unten zulässt, um die Vorderkante nach unten entlang der Außenfläche des Trokars zu treiben, wobei gleichzeitig die Hinterkante auf die Außenfläche des Trokars zu und in Kontakt mit dieser gebracht wird, wobei optional der Griff eine Länge aufweist, die größer als die Länge der bogenförmigen Passfläche ist.

3. Vorrichtung nach Anspruch 2, wobei der Griff (120) eine obere Oberfläche umfasst, die dazu bemessen ist, den Daumen eines Benutzers aufzunehmen.

4. Vorrichtung nach einem der vorangehenden Ansprüche, wobei sich die bogenförmige Passfläche betriebsfähig nur teilweise um den Trokar oder den anderen Körper erstreckt, wobei optional die bogenförmige Passfläche einen Bogen definiert, der von weniger als 180 Grad eingeschlossen wird.

5. Vorrichtung nach einem der Ansprüche 1 bis 3, wobei die bogenförmige Passfläche einen Bogen definiert, der größer als oder gleich 180 Grad ist.

6. Vorrichtung nach einem der vorangehenden Ansprüche, wobei die bogenförmige Passfläche dazu konfiguriert ist, mit dem Trokar oder dem anderen Körper schnappend in Eingriff zu treten.

7. Vorrichtung nach einem der Ansprüche 1 bis 3, wobei die bogenförmige Passfläche dazu konfiguriert ist, sich vollständig um den Trokar oder den anderen Körper herum zu erstrecken, wobei optional die bogenförmige Passfläche dazu konfiguriert ist, gegen den Trokar oder den anderen Körper gespannt zu werden.

8. Vorrichtung nach einem der vorangehenden Ansprüche, wobei der Nadelführungskanal einen offenen Abschnitt umfasst, in dem sich ein Nadeltreiber während eines Hindurchgelangens des Nadeltreibers durch die Vorrichtung betriebsfähig aus der Vorrichtung heraus erstreckt.

9. Vorrichtung nach einem der vorangehenden Ansprüche, wobei die bogenförmige Passfläche eine Kappe (116) definiert, in der ein Finger oder ein anderer Digitus aufgenommen werden kann.

10. Vorrichtung nach einem der vorrangehenden Ansprüche, umfassend eine Außenfläche (140), die konturiert ist, um mindestens eine verschobene Anlagefläche (141) zu definieren, die in Gebrauch die Bewegung der Vorrichtung relativ zur Bauchdecke, in der sie sich befindet, einschränkt.

11. Vorrichtung nach Anspruch 10, wobei die Anlagefläche (140) ein Verankern der Vorrichtung in der Bauchdecke bereitstellt.

12. Vorrichtung nach Anspruch 10, wobei die Außenfläche nach innen zu einer Vorderkante der Vorrichtung hin verjüngt ist, um ein verjüngtes Eintrittsglied bereitzustellen, um das Einführen der Vorrichtung in die Bauchdecke leichter zu machen.

13. Vorrichtung nach Anspruch 12, wobei die Vorderkante mit einem Innenradius abgeschrägt ist, um das Hängenbleiben an abgestuften oder mit Gewinde versehenen Trokarschäften zu verhindern.

14. Vorrichtung nach einem der vorangehenden Ansprüche, wobei die Eintrittsöffnung relativ zu dem Griff außermittig ist, um zu erleichtern, dass die Fadenzuführvorrichtung dem Nadelführungskanal dargeboten wird.

15. Vorrichtung nach Anspruch 2, wobei der Griff eine Länge aufweist, die ausreicht, um das Positionieren des Griffs in der Handfläche der Hand eines Benutzers zuzulassen.

## Revendications

1. Dispositif chirurgical (100) pour un accouplement avec un trocart (730) or un autre corps, le dispositif comprenant :
a. un bord d'attaque (105),
b. un bord de fuite (110),
c. une surface d'appariement arquée (115) fournie entre les bords d'attaque et de fuite, la surface d'appariement arquée étant de manière fonctionnelle en contact intime avec une surface externe du trocart ou de l'autre corps,
d. seulement un canal de guidage d'aiguille (130), le canal de guidage d'aiguille comprenant un orifice d'entrée (131) pour recevoir un dispositif de pose de sutures (700) et un orifice de sortie (132) à partir duquel le dispositif de pose de sutures peut être éloigné du dispositif (100), les orifices d'entrée et de sortie pour le canal de guidage d'aiguille étant fournis sur le même côté de la surface d'appariement arquée, le canal de guidage d'aiguille définissant une trajectoire arquée ayant une surface convexe à proximité de la surface d'appariement arquée de sorte que la suture sortira de la surface d'appariement arquée en étant déplacée hors du canal de guidage d'aiguille.

2. Dispositif selon la revendication 1 comprenant une poignée (120) qui permet l'application d'une force de rotation vers le bas pour entraîner le bord d'attaque vers le bas le long de la surface externe du trocart amenant en même temps le bord de fuite vers et en contact avec la surface externe du trocart, facultativement dans lequel la poignée a une longueur supérieure à la longueur de la surface d'appariement arquée.

3. Dispositif selon la revendication 2 dans lequel la poignée (120) comprend une surface supérieure dimensionnée pour recevoir le pouce d'un utilisateur.

4. Dispositif selon l'une quelconque des revendications précédentes dans lequel la surface d'appariement arquée s'étend de manière fonctionnelle partiellement seulement autour du trocart ou de l'autre corps, facultativement dans lequel la surface d'appariement arquée définit un arc sous-tendu par moins de 180 degrés.

5. Dispositif selon l'une quelconque des revendications 1 à 3 dans lequel la surface d'appariement arquée définit un arc supérieur ou égal à 180 degrés.

6. Dispositif selon l'une quelconque des revendications précédentes dans lequel la surface d'appariement arquée est configurée pour s'encliqueter avec le trocart ou l'autre corps.

7. Dispositif selon l'une quelconque des revendications 1 à 3 dans lequel la surface d'appariement arquée est configurée pour s'étendre pleinement autour du trocart ou de l'autre corps, facultativement dans lequel la surface d'appariement arquée est configurée pour être tendue contre le trocart ou l'autre corps.

8. Dispositif selon l'une quelconque des revendications précédentes dans lequel le canal de guidage d'aiguille comprend une partie ouverte au sein de laquelle un dispositif d'entraînement d'aiguille s'étendra de manière fonctionnelle hors du dispositif pendant un passage du dispositif d'entraînement d'aiguille à travers le dispositif.

9. Dispositif selon l'une quelconque des revendications précédentes dans lequel la surface d'appariement arquée définit un culot (116) au sein duquel un doigt ou un pouce peut être reçu.

10. Dispositif selon l'une quelconque des revendications précédentes comprenant une surface externe (140) profilée pour définir au moins une surface de butée déplacée (141) qui pendant l'utilisation restreindra le mouvement du dispositif par rapport à la paroi abdominale au sein de laquelle il est situé.

11. Dispositif selon la revendication 10 dans lequel la surface de butée (140) fournit un ancrage du dispositif au sein de la paroi abdominale.

12. Dispositif selon la revendication 10 dans lequel la surface externe est effilée vers l'intérieur vers un bord d'attaque du dispositif pour fournir un élément d'entrée effilé pour faciliter l'insertion du dispositif jusque dans la paroi abdominale.

13. Dispositif selon la revendication 12 dans lequel le bord d'attaque est chanfreiné ayant un rayon interne pour éviter qu'il ne s'accroche sur les manches à gradins ou filetés de trocarts.

14. Dispositif selon l'une quelconque des revendications précédentes dans lequel le port d'entrée est excentré par rapport à la poignée pour faciliter la présentation du dispositif de pose de sutures au canal de guidage d'aiguille.

15. Dispositif selon la revendication 2 dans lequel la poignée a une longueur suffisante pour permettre le positionnement de la poignée dans la paume de main d'un utilisateur.
